# FASCICULE DE BREVET EUROPEEN

(11) **EP 1 875 893 B1**
(45) Date de publication et mention de la délivrance du brevet: **20.10.2010**
(21) Numéro de dépôt: 07301198.3
(22) Date de dépôt: 02.07.2007
(51) Int. Cl.: A61K 8/60, A61K 8/81, A61Q 1/00, A61Q 19/00

(54) **Composition cosmétique associant un dérivé C-glycoside à un polymère associatif**
Kosmetische Zusammensetzung zwischen einem C-Glykosid-Derivat und einem assoziativen Polymer
Composition combining a C-glycoside derivative with an associative polymer

(30) Priorité: 03.07.2006 FR 0606021
(43) Date de publication de la demande: 09.01.2008
(73) Titulaire: L'Oréal, 75008 Paris (FR)
(72) Inventeur: WILLEMIN, Claudie, 75008, PARIS (FR); CHEVALIER, Véronique, 94440, VILLECRESNES (FR)
(74) Mandataire: Le Coupanec, Pascale A.M.P.

(56) Documents cités:
- EP-A- 1 473 024
- WO-A2-02/051828

## Description

La présente invention concerne des compositions cosmétiques et/ou dermatologiques comprenant un dérivé C-glycoside et un polymère associatif, ainsi que l'utilisation de ces compositions dans un procédé de traitement des matières et/ou des fibres kératiniques.

Plus particulièrement, les compositions de l'invention sont destinées au soin et/ou au maquillage des matières et/ou des fibres kératiniques.

Au sens de l'invention, on entend désigner par « matières et/ou fibres kératiniques », par exemple, la peau, les muqueuses, les lèvres, le cuir chevelu, les cils, les sourcils et les cheveux.

Les sucres et dérivés du sucre sont des produits déjà mis à profit à des fins diverses pour la formulation de compositions cosmétiques destinées tant au soin de la peau qu'au soin et/ou lavage des fibres kératiniques.

Ainsi, dans WO 99/24009, le D-xylose et ses dérivés sont proposés à des fins de préparation de produits cosmétiques ou pharmaceutiques visant à améliorer la fonctionnalité des cellules de l'épiderme.

Parmi les sucres utilisables dans le domaine, les dérivés C-glycosides s'avèrent tout particulièrement intéressants. Ainsi, certains dérivés C-glycosides ont démontré des propriétés biologiques intéressantes, en particulier pour lutter contre le vieillissement de l'épiderme et/ou contre le dessèchement de la peau. De tels composés sont notamment décrits dans le document WO 02/051828.

Ces composés sont plus particulièrement représentés par la formule : 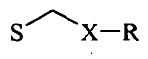 dans laquelle S représente un monosaccharide ou un polysaccharide, R représente différents radicaux linéaires ou cycliques et le groupement X peut représenter un groupement choisi parmi : -CO-, -CH(NR₁R₂)-, CHR'-, -C(=CHR')- avec R₁, R₂ et R' pouvant représenter différent radicaux, dont le radical hydroxyle pour R₁ et R₂.

Par ailleurs on recherche également à disposer de compositions cosmétiques qui puissent s'appliquer de manière satisfaisante sur les matières kératiniques et à cette fin, ces compositions doivent présenter des textures adaptées pour permettre une bonne application (notamment un bon étalement) sur les matières kératiniques.

Malheureusement, l'introduction des dérivés C-glycosides précédemment cités dans une formulation cosmétique aqueuse peut se traduire par une diminution non négligeable de la viscosité, induisant ainsi une fluidification importante de la composition.

Une composition trop fluide est difficile à appliquer sur les matières kératiniques. Une telle composition s'écoule des matières kératiniques, notamment de la peau, sur lesquelles elle est appliquée. Son application sur les matières kératiniques que l'on souhaite traiter manque de précision et rend ainsi son usage peu attractif.

En outre, la présence d'un dérivé C-glycoside s'avère affecter le pouvoir épaississant de certains agents gélifiants conventionnels.

Les solutions alternatives consistant à suppléer à cette baisse de viscosité par l'ajout de cire(s) et/ou d'alcool gras ne s'avèrent pas satisfaisantes. Les formulations ainsi obtenues sont généralement trop épaisses et blanchissantes, difficiles à appliquer, et notamment à étaler, sur les matières kératiniques, et confèrent une sensation de lourdeur, de difficulté à pénétrer lors de l'application sur la peau.

Ainsi, il existe un besoin de disposer de compositions cosmétiques, dermatologiques ou thérapeutiques comprenant des dérivés C-glycosides et qui puissent néanmoins être dotées d'un épaississement significatif si besoin est.

Il existe également un besoin de disposer de compositions cosmétiques ou thérapeutiques présentant une viscosité appropriée à une application facile sur les matières et/ou fibres kératiniques et notamment des compositions fluides à haute teneur en particules demeurant facilement vaporisables.

La présente invention a précisément pour objet de satisfaire à ces besoins.

Plus précisément, la présente invention concerne une composition cosmétique et/ou pharmaceutique, notamment dermatologique, comprenant, dans un milieu physiologiquement acceptable contenant un milieu aqueux, au moins un dérivé C-glycoside et au moins un polymère associatif.

De manière surprenante, les inventeurs ont observé que l'addition d'un dérivé C-glycoside à une composition comprenant un polymère associatif n'affectait pas significativement la viscosité de ladite composition et permettait ainsi de formuler celle-ci sous une forme appropriée à sa manipulation lors de son application.

Qui plus est, cette association spécifique C-glycoside-polymère associatif permet également de conférer à une composition de l'invention un aspect agréable, et lors de son application, des propriétés de sensation confortable.

Selon encore un autre de ses objets, la présente invention concerne un procédé de traitement non thérapeutique ou de maquillage des matières et/ou des fibres kératiniques comprenant au moins l'étape d'appliquer sur lesdites matières et/ou fibres kératiniques au moins une couche d'une composition cosmétique conforme à l'invention.

### POLYMERE ASSOCIATIF

Les polymères associatifs sont des polymères hydrosolubles capables, dans un milieu aqueux, de s'associer réversiblement entre eux ou avec d'autres molécules. Leur structure chimique comprend des zones hydrophiles, et des zones hydrophobes caractérisées par au moins une chaîne grasse.

Les polymères associatifs selon l'invention peuvent être de type anionique, ou non ionique.

### a) Polymères associatifs de type anionique

Parmi les polymères associatifs de type anionique, on peut citer :
- les co-polymères comportant au moins un motif hydrophile de type acide carboxylique insaturé oléfinique, et au moins un motif hydrophobe de type ester d'alkyle (C₁₀-C₃₀) d'acide carboxylique insaturé.

Ces polymères sont choisis parmi ceux dont le motif hydrophile de type acide carboxylique insaturé oléfinique correspond au monomère de formule (II) suivante : 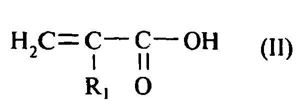 dans laquelle, R₁ désigne H ou CH₃ ou C₂H₅, c'est-à-dire des motifs acide acrylique, acide méthacrylique ou acide éthacrylique,
et dont le motif hydrophobe de type ester d'alkyl (C₁₀-C₃₀) d'acide carboxylique insaturé correspond au monomère de formule (III) suivante : 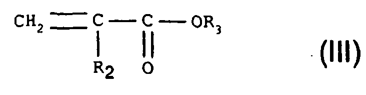 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

On peut mentionner en particulier les co-polymères d'acides carboxyliques insaturés et de carboxylates insaturés en C₁₀-C₃₀.

Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Parmi ce type de polymères associatifs anioniques, on utilisera plus particulièrement des polymères formés à partir d'un mélange de monomères comprenant :
(i) essentiellement de l'acide acrylique,
(ii) un ester de formule (III) décrite ci-dessus et dans laquelle R₂ désigne H ou CH₃, R₃ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyle, le divinylbenzène, le diméthacrylate de (poly)éthylèneglycol, et le méthylène-bis-acrylamide.

Parmi ce type de polymères associatifs anioniques, on utilisera plus particulièrement ceux constitués de 95 à 60 % en poids d'acide acrylique (motif hydrophile), 4 à 40 % en poids d'acrylate d'alkyles en C₁₀-C₃₀ (motif hydrophobe), et 0 à 6 % en poids de monomère polymérisable réticulant, ou bien ceux constitués de 98 à 96 % en poids d'acide acrylique (motif hydrophile), 1 à 4 % en poids d'acrylate d'alkyles en C₁₀-C₃₀ (motif hydrophobe), et 0,1 à 0,6 % en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

Les terpolymères acryliques obtenus à partir d'acide méthacrylique comme composant (a), d'acrylate de méthyle comme composant (b) et d'un macromonomère uréthane non-ionique comme composant (c), ayant la structure (III) suivante : 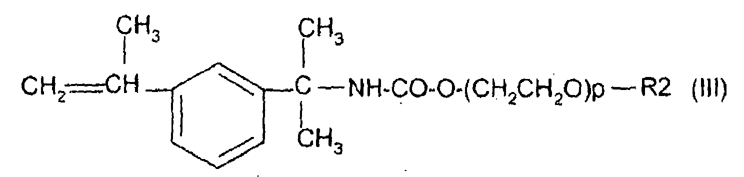 dans laquelle p va de 6 à 150 et R2 est choisi parmi les radicaux alkyle linéaires comportant de 18 à 26 et de préférence de 20 à 24 atomes de carbone. De préférence, le radical R2 dans la composé de formule (III) est un radical d'origine végétale, tel que le radical béhényle.

Les terpolymères utilisés selon l'invention sont généralement en dispersion aqueuse.

Convient tout particulièrement à l'invention le terpolymère acide méthacrylique / acrylate de méthyle / diméthyl métaisopropényl benzyl isocyanate d'alcool béhényle éthoxylé (40 OE) en dispersion aqueuse à 25 % en poids vendu sous la dénomination VISCOPHOBE DB 1000^{®} par la société AMERCHOL.

### d) Polymères associatifs de type non ionique

Selon l'invention, les polymères associatifs de type non ioniques sont choisis parmi :
- les polyéthers-polyuréthanes comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques,

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéther-polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéther-polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéther-polyuréthanes non-ioniques à chaîne grasse utilisables dans l'invention, on peut aussi utiliser le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple de tels polymères, on peut citer le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéther-polyuréthanes utilisables selon l'invention sont en particulier ceux décrits dans l'article de G. Fonnum, J. Bakke et Fk. Hansen - Colloid Polym. Sci 271, 380-389 (1993).

De tels polyéther-polyuréthanes sont vendus notamment par la société ROHM & HAAS sous les appellations Aculyn 46^{®} et Aculyn 44^{®}; l'ACULYN 46^{®} est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4 %) et d'eau (81 %) ; l'ACULYN 44^{®} est un polycondensat de polyéthylèneglycol à 150 ou 180 moles d'oxyde d'éthylène, d'alcool décylique et de méthylène bis(4-cyclohexylisocyanate) (SMDI), à 35 % en poids dans un mélange de propylèneglycol (39 %) et d'eau (26 %).

Selon un mode de réalisation, une composition de l'invention comprend au moins environ de 0,01 à 30 % en poids, en particulier d'environ 0,05 à 15 % en poids, de préférence d'environ 0,1 à 10 % en poids et plus particulièrement d'environ 0,1 à 5% en poids de polymère(s) associatif(s) par rapport au poids total de la composition

### DERIVES C-GLYCOSIDES

Un dérivé C-glycoside convenant à l'invention peut être un composé de formule générale (I) suivante : 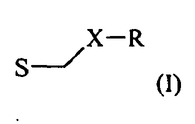 dans laquelle :
- R désigne un radical linéaire en C₁-C₄ ;
- X représente un groupement -CH(OH)-,
- S représente le D-xylose, et la liaison S-CH₂-X représente une liaison de nature C-aromérique, qui peut être α ou β ainsi que leurs sels cosmétiquement acceptables, leurs solvates tels que les hydrates.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Parmi les dérivés C-glycoside de formule (I), utilisés selon l'invention, on considère tout particulièrement :
C-β-D-xylopyranoside-2-hydroxy-propane ;
C-α-D-xylopyranoside-2-hydroxy-propane ;

Selon un mode de réalisation, on peut utiliser le C-β-D-xylopyranoside-2-hydroxy-propane sous la forme d'une solution à 30 % en poids en actifs dans un mélange eau/1,2 propane diol 60/40, tel que celui commercialisé sous le nom de MEXORYL SBB^{®} par la société CHIMEX.

Bien entendu, selon l'invention, un dérivé C-glycoside répondant à la formule (I) peut être utilisé seul ou en mélange avec d'autres dérivés C-glycosides et en toutes proportions.

Un dérivé de C-glycoside convenant à l'invention peut notamment être obtenu par la méthode de synthèse décrite dans le document WO 02/051828.

La quantité de dérivé C-glycoside à mettre en oeuvre dans une composition selon l'invention dépend de l'effet cosmétique ou thérapeutique recherché, et peut donc varier dans une large mesure.

L'homme de l'art peut aisément, sur la base de ses connaissances générales, déterminer les quantités appropriées.

Une composition selon l'invention peut comprendre un dérivé de C-glycoside à raison d'environ 0,0001 % à environ 25% en poids par rapport au poids total de la composition, et en particulier d'environ 0,001 % à environ 10% en poids, et plus particulièrement d'environ 0,05 à 5% en poids de matière active de dérivé C-glycoside par rapport au poids total de la composition.

Selon un mode de réalisation, une composition selon l'invention peut comprendre au moins un dérivé de C-glycoside et au moins un polymère associatif en particulier de type anionique et notamment de type terpolymère acrylique et plus particulièrement tel que défini ci-dessus, selon un rapport pondéral variant d'environ d'environ 0.002 à environ 50, et plus particulièrement d'environ 0.01 à environ 15.

### PROTOCOLE DE MESURE DE LA VISCOSITE

La viscosité d'une composition de l'invention peut être mesurée selon tout procédé connu de l'homme de l'art, et notamment selon le procédé conventionnel suivant. Ainsi, la mesure peut être réalisée à 25 °C à l'aide d'un Contraves TV ou Rhéomat 180, équipé d'un mobile tournant à 200t/mn. L'homme du métier peut choisir le mobile permettant de mesure la viscosité, parmi les mobiles, M1 ou M2 sur la base de ses connaissances générales, de manière à pouvoir réaliser la mesure.

L'ajout d'un C-glycoside tel que défini précédemment à une solution aqueuse comprenant un polymère associatif selon l'invention se traduit par une variation de viscosité inférieure à 20% mesurée par rapport à la viscosité d'une solution comprenant uniquement le polymère, en particulier inférieure ou égale à 15%, en particulier inférieure ou égale à 10%.

Une composition selon l'invention comprend un milieu physiologiquement acceptable.

Par « milieu physiologique acceptable », on entend désigner un milieu compatible avec les matières et/ou les fibres kératiniques d'êtres humains, comme par exemple, de manière non limitative, la peau, les muqueuses, les ongles, le cuir chevelu et/ou les cheveux.

Ce milieu physiologiquement acceptable comprend une phase aqueuse, éventuellement en mélange ou non avec un ou plusieurs solvants organiques tels qu'un alcool en C₁-C₈, notamment l'éthanol, l'isopropanol, le tert-butanol, le n-butanol, des polyols comme la glycérine, le propylène glycol, le butylèneglycol, et des éthers de polyol.

Une composition selon l'invention peut également comprendre une phase grasse, qui peut comprendre des huiles, des gommes, des cires usuellement utilisées dans le domaine d'application considéré.

Ainsi selon un mode de réalisation une composition selon l'invention peut en outre comprendre au moins une phase grasse choisie parmi une phase grasse solide à température ambiante (20-25 °C) et pression atmosphérique et/ou une phase grasse liquide à température ambiante (20-25 °C) et pression atmosphérique.

Une phase grasse liquide convenant à la mise en oeuvre de l'invention peut comprendre une huile volatile, une huile non volatile, et un mélange de celles-ci. Une huile volatile ou non volatile peut être une huile hydrocarbonée, notamment d'origine animale ou végétale, une huile synthétique, une huile siliconée, une huile fluorée ou un mélange de celles-ci.

Une phase grasse solide convenant à la mise en oeuvre de l'invention peut être, par exemple, choisie parmi les corps gras pâteux, les gommes, et leurs mélanges.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Lorsqu'une composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique.

Un émulsionnant et un coémulsionnant peuvent être présents dans une composition de l'invention en une proportion allant de 0,3 % à 30 % en poids, et en particulier de 0,5 à 20 % en poids par rapport au poids total de la composition.

Une émulsion selon l'invention peut, en outre, contenir des vésicules lipidiques.

Lorsqu'une composition selon l'invention est une solution ou un gel huileux, la phase grasse peut représenter plus de 90 % du poids total de la composition.

Une composition selon l'invention peut contenir également des adjuvants habituels dans le domaine considéré, tels que des tensioactifs, des émulsionnants, des gélifiants hydrophiles ou lipophiles, des additifs hydrophiles ou lipophiles, des conservateurs, des antioxydants, des solvants, des parfums, des charges, des filtres UVA et ou UVB (organique ou inorganique, solubles ou insolubles), des pigments, des fibres, des agents chélateurs, des absorbeurs d'odeur, des matières colorantes, et d'autres actifs cosmétiques ou pharmaceutiques.

Les quantités de ces différents adjuvants sont celles classiquement utilisées dans le domaine cosmétique, et peuvent être par exemple varier de 0,01 % à 30 % du poids total de la composition. De manière générale, les quantités sont ajustées en fonction de la formulation réalisée. Ces adjuvants, selon leur nature, peuvent être introduits dans la phase grasse, dans la phase aqueuse et/ou dans les sphérules lipidiques.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, polyéthylène.

Une composition de l'invention peut se présenter sous toutes les formes galéniques envisageables.

Notamment, une composition selon l'invention peut avoir la forme d'une solution aqueuse, hydroalcoolique; d'une dispersion du type lotion ou sérum; d'une émulsion eau-dans-huile, huile-dans-eau ou multiple; d'une suspension; de microcapsules ou microparticules; de dispersions vésiculaires de type ionique et/ou non ionique; d'une lotion aqueuse, ou sous forme de sérum; de capsules, de granulés, de sirops, de comprimés; de mousse, de préparation solide; de composition pour aérosol comprenant également un agent propulseur sous pression.

Une composition selon l'invention peut se présenter sous la forme d'une composition pour soin capillaire, notamment un shampooing, une lotion de mise en plis, une lotion traitante, une crème ou un gel coiffant, une composition de teinture, notamment d'oxydation, des lotions restructurantes pour les cheveux, une composition de permanente (notamment une composition pour le premier temps d'une permanente), une lotion ou un gel antichute, un shampooing antiparasitaire.

Elle peut également se présenter sous la forme d'une composition de nettoyage, de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, comme des lotion de nettoyage, composition de bronzage artificiel); une composition de maquillage du corps ou du visage telle qu'un fond de teint; une composition pour le bain; une composition désodorisante comprenant par exemple un agent bactéricide; une composition après-rasage; une composition épilatoire; une composition contre les piqûres d'insectes; une composition anti-douleur ; une composition dermatologique ou pharmaceutique pour traiter certaines maladies de la peau comme l'eczéma, la rosacée, le psoriasis, les lichens, les prurits sévères.

Lorsqu'une composition selon l'invention est destinée à un usage de type peeling, elle peut également se présenter sous toutes les formes galéniques évoquées ci-dessus pour autant qu'elle s'élimine facilement par rinçage, et notamment sous forme de gel aqueux, de solution aqueuse ou hydroalcoolique.

Une composition selon l'invention peut être appliquée par tout moyen permettant une répartition uniforme et notamment à l'aide d'un coton, d'une tige, d'un pinceau, d'une gaze, d'une spatule ou d'un tampon, ou encore par pulvérisation, et peut être éliminée par rinçage à l'eau ou à l'aide d'un détergent doux.

Une composition selon l'invention peut se présenter sous une forme fluide de type liquide vaporisable ou non, sous forme de pâte, d'émulsion directe ou inverse, ou de gel ou de support imprégné.

En particulier, une composition selon l'invention peut se présenter sous une forme solide, notamment compacte, pulvérulente ou coulée ou sous une forme de stick.

Une composition selon l'invention peut également se présenter sous la forme d'un produit de soin, d'un produit solaire ou après solaire, d'un produit de soin de photo-protection quotidienne, d'un produit pour le corps, d'un fond de teint à appliquer sur le visage ou sur le cou, d'un produit anti-cernes, d'un correcteur de teint, d'une crème teintée ou d'une base de maquillage pour le maquillage pour le visage ou d'une composition de maquillage pour le corps.

Une composition selon l'invention peut en outre comprendre un ou des actif(s) cosmétique(s) ou thérapeutique(s) additionnel(s).

Ainsi une composition selon la présente invention peut en particulier comprendre au moins :
- un agent anti-âge et/ou antiride,
- un agent anti-glycation
- un inhibiteur de NO-synthase,
- un agent agissant sur les macromolécules dermiques ou épidermiques et/ou empêchant leur dégradation,
- un agent modulant la prolifération des fibroblastes ou des kératinocytes et/ou la différenciation des kératinocytes et,
- un agent myorelaxant,
- un agent dépigmentant ou pro-pigmentant,
- un agent anti-microbien,
- un agent tenseur,
- un agent anti-pollution ou anti-radicalaire,
- un agent apaisant,
- un actif lipolytique ou ayant une activité favorable, directe ou indirecte, sur la diminution du tissu adipeux,
- un agent agissant sur la microcirculation et,
- un agent anti-acné,
- un agent hydratant.

Une composition selon l'invention peut être mise en oeuvre à des fins d'amélioration de l'état général d'un épiderme, en particulier de la peau, et notamment pour le maintient ou la restauration de ses fonctions physiologiques et/ou de son aspect esthétique.

Ainsi, une composition selon l'invention peut être avantageusement mise en oeuvre afin de lutter contre le vieillissement de l'épiderme, maintenir et/ou stimuler l'hydratation et/ou lutter contre le dessèchement de la peau, améliorer la tonicité de la peau, maintenir ou restaurer la souplesse et l'élasticité de la peau, améliorer la minéralisation de l'épiderme, améliorer la vitalité de l'épiderme, faciliter les échanges inter-cellulaires, et lutter contre les gerçures et l'aspect craquelé de la peau.

Une composition selon l'invention peut être destinée à une application cosmétique et/ou dermatologique.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### EXEMPLES

Dans les exemples figurant ci-après, le dérivé C-glycoside est le C-β-D-xylopyranoside-2-hydroxy-propane. Il s'agit plus particulièrement d'une solution aqueuse à 30 % en poids de matière active (MA) dans un mélange eau/proplylène glycol 60/40 (vendu sous la dénomination MEXORYL SBB^{®} par la société CHIMEX).

### Exemples 1 à 3

On a réalisé un 3 gels aqueux avec 3 polymères épaississants différents (Polymère associatif selon l'invention et 2 polymères non associatifs hors invention, et pour chacun des polymère le gel été réalisé avec ou en l'absence de C-β-D-xylopyranoside-2-hydroxy-propane. On a ensuite mesuré la viscosité des gels aqueux obtenus après 24 heures de stockage à température ambiante (viscosité mesurée à 25°C à l'aide d'un Contraves TV mobile M3 après 10 minutes de rotation à 200tours/minutes

| | **Exemple 1A** | **Exemple 1B (invention)** |
|---|---|---|
| C -β -D-xylopyranoside-2-hydroxypropane en solution à 30 % en poids dans un mélange eau/propylèneglycol 60/40 | 0 | 2,98 % soit 1% MA |
| terpolymère acide méthacrylique / acrylate de méthyle / diméthyl metaisopropényl benzyl isocyanate d'alcool béhényle éthoxylé (40 OE) (VISCOPHOBE DB 1000 d'AMERCHOL) | 2 % | 2 % |
| Soude | 1,5 % | 1,5 % |
| Eau | Qsp 100 % | Qsp 100 % |
| Viscosité (mpa.s) | 5,1 | 4,4 |

| | **Exemple 2A** | **Exemple 2B TEMOIN** |
|---|---|---|
| C -β -D-xylopyranoside-2-hydroxypropane en solution à 30 % en poids dans un mélange eau/propylèneglycol 60/40 | 0 | 2,98 % soit 1% MA |
| COPOLYMERE ACRYLAMIDE/ACRYLAMIDO 2-METHYL PROPANE SULFONATE DESODIUM EN EMULSION INVERSE A 40% DANS ISOPARAFFINE/EAU (Sépigel 305 de chez SEPPIC) | 3% | 3 % |
| Eau | Qsp 100 % | Qsp 100 % |
| Viscosité (pa.s) | 3,16 | 2,07 |

| | **Exemple 3A** | **Exemple 3B** **TEMOIN** |
|---|---|---|
| C -β -D-xylopyranoside-2-hydroxypropane en solution à 30 % en poids dans un mélange eau/propylèneglycol 60/40 | 0 | 2,98 % soit 1% MA |
| Polymère carboxyvinylique (Carbopol 981 de chez NOVEON) | 3 % | 3 % |
| Eau | Qsp 100 % | Qsp 100 % |
| Viscosité (pa.s) | 9,5 | 7,2 |

Ces essais montrent que seul le polymère associatif Viscophobe DB1000 en présence du C -β -D-xylopyranoside-2-hydroxy-propane présente une faible variation de viscosité. Ainsi, la présence du polymère associatif permet de maintenir la viscosité du gel aqueux en présence du C -β -D-xylopyranoside-2-hydroxy-propane.

### Exemple 4 : Fluide aux esters de sucres

| Composés | % |
|---|---|
| SESQUI-STEARATE DE METHYL GLUCOSE (TEGO CARE PS de GOLDSCHMIDT) | 2 |
| MELANGE D'ALCOOL STEARYLIQUE ET CETHYSTEARYLIQUE (Promulgen G de Noveon) | 2 |
| CYLOHEXA-DIMETHYLSILOXANE | 10 |
| SHOREA ROBUSTA (BEURRE DE SALE de Stéarine Dubois) | 6 |
| HUILE D'AMANDES D'ABRICOTS | 2 |
| SESQUISTEARATE DE METHYL-GLUCOSE OXYETHYLENE (GLUCAMATE SSE 20 de Noveon) | 2 |
| Terpolymère acide méthacrylique / acrylate de méthyle / diméthyl métaisopropényl benzyl isocyanate d'alcool béhényle éthoxylé (40 OE) (VISCOPHOBE DB 1000 d'AMERCHOL) | 1 |
| CONSERVATEUR | 0,5 |
| EAU | Qsp 100 |
| C-β-D-XYLOPYRANOSIDE-2-HYDROXY-PROPANE EN SOLUTION A 30 % EN POIDS DANS UN MELANGE EAU/PROPYLENEGLYCOL 60/40 | 1 % MA |

### Exemple 5 : Crème anti-âge

| Composés | % |
|---|---|
| MELANGE MONO/DISTEARATE DE GLYCERYLE / STEARATE DE POLYETHYLENE GLYCOL (100 OE) (ARLACEL 135 de chez Uniqema) | 2,5 |
| MYRJ 53 | 2,5 |
| ALCOOL CETYLIQUE | 1 |
| ALCOOL STEARYLIQUE | 1 |
| ISOPARAFFINE (6-8 MOLES D'ISOBUTYLENE) HYDROGENEE (PARLEAM de chez NOF Corporation) | 5 |
| CYCLOPENTASILOXANE | 15 |
| EAU | QSP 100 |
| CONSERVATEUR | 0,5 |
| Terpolymère d'ACIDE METHACRYLIQUE/METHYL ACRYLATE/ DIMETHYL META-ISOPROPENYL BENZYL ISOCYANATE D'ALCOOL ETHOXYLE (Viscophobe DB 1000 d'Amercol) | 0,3 |
| C-β-D-XYLOPYRANOSIDE-2-HYDROXY-PROPANE EN SOLUTION A 30 % EN POIDS DANS UN MELANGE EAU/PROPYLENEGLYCOL 60/40 | 5% MA |

### Exemple 6 : Formule solaire fluide vaporisable

| COMPOSES | Ex A | Ex B |
|---|---|---|
| SEQUESTRANT | Qs | Qs |
| TRIETHANOLAMINE | Qs | Qs |
| BUTYLMETHOXYDIBENZYLMETHANE 4-TERTIOBUTYL-4'-METHOXY-DIBENZOYLMETHANE (Parsol 1789 de DSM Nutritional product) | 1 | 3 |
| SALICYLATE de 2- ETHYLHEXYL (Néohéliopan OS de Symrise) | 0 | 5 |
| ETHYLHEXYL TRIAZONE (Uvinul T 150 de BASF) | 0,5 | 0,5 |
| TEREPHTHALYLIDENE DICAMPHOR SULFONIC ACID (Mexoryl SX de CHIMEX) | 1,5 | 1,5 |
| OCTOCRYLENE (Uvinul N 539 de BASF) | 10 | 10 |
| TITANIUM DIOXYDE (MT100 AQ de Tayca) | 0 | 3,5 |
| COPOLYMERE DIGLYCOL CYCLOHEXANEDIMETHANOL ISOPHTALATES SULFOISOPHTALATES (EASTMAN AQ 38S POLYMER de chez Eastman Chemical) | 2 | 2 |
| TERPOLYMERE d'ACIDE METHACRYLIQUE/METHYL ACRYLATE/ DIMETHYL META-ISOPROPENYL BENZYL ISOCYANATE D'ALCOOL ETHOXYLE (Viscophobe DB 1000 d'Amercol) | 0,5 | 0,5 |
| CYCLOHEXASILOXANE | 5 | 5 |
| EAU | Qsp 100 | Qsp 100 |
| C₁₂₋C₁₅ ALKYL BENZOATE (FINSOLV TN de chez FINETEX) | 6 | 1 |
| GLYCERINE | 3 | 3 |
| C-β-D-XYLOPYRANOSIDE-2-HYDROXY-PROPANE EN SOLUTION A 30 % EN POIDS DANS UN MELANGE EAU/PROPYLENEGLYCOL 60/40 (Mexoryl SBB) | 3 % MA | 3 % MA |
| CONSERVATEUR | Qs | Qs |

## Revendications

1. Composition cosmétique et/ou pharmaceutique, notamment dermatologique, comprenant, dans un milieu physiologiquement acceptable, un milieu aqueux, au moins un dérivé C-glycoside et au moins un polymère associatif, ledit dérivé C-glycoside répondant à la formule générale (I) suivante : 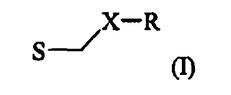 dans laquelle :
R désigne un radical linéaire en C₁-C₄,
X représente un groupement -CH(OH)-, et
S représente le D-xylose, et
- la liaison S-CH₂-X représente une liaison de nature C-anomérique, qui peut être a ou β,
ainsi que leurs sels cosmétiquement acceptables, leurs solvates tels que les hydrates, et ledit polymère associatif étant choisi parmi :
- un polymère associatif anionique choisi parmi :
- les co-polymères comportant au moins un motif hydrophile de type acide carboxylique insaturé oléfinique, et au moins un motif hydrophobe de type ester d'alkyle (C₁₀-C₃₀) d'acide carboxylique insaturé, ces polymères étant tels que le motif hydrophile de type acide carboxylique insaturé oléfinique correspond au monomère de formule (II) suivante : 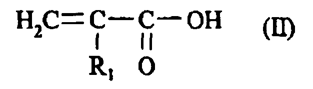 dans laquelle, R₁ désigne H ou CH₃ ou C₂H₅, par exemple des motifs acide acrylique, acide méthacrylique ou acide éthacrylique,
et dont le motif hydrophobe de type ester d'alkyl (C₁₀-C₃₀) d'acide carboxylique insaturé correspond au monomère de formule (III) suivante : 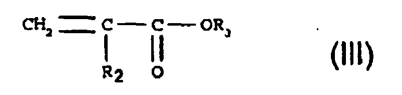 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂,
- les terpolymères acryliques obtenus à partir d'acide méthacrylique (a), d'acrylate de méthyle (b) et d'un macromonomère uréthane non-ionique (c), ayant la structure (III) suivante : 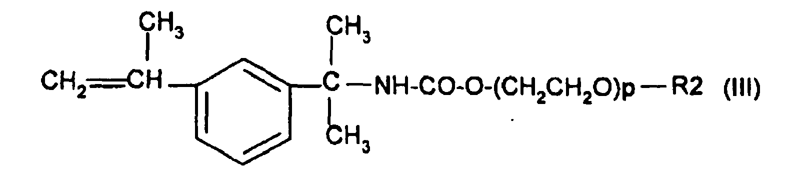
dans laquelle p va de 6 à 150 et R2 est choisi parmi les radicaux alkyle linéaires comportant de 18 à 26 et de préférence de 20 à 24 atomes de carbone, le radical R₂ dans la composé de formule (III) étant de préférence un radical d'origine végétale, tel que le radical béhényle, et
- un polymère associatif non-ionique choisi parmi les polyéther-polyuréthanes comportant des séquences hydrophiles et au moins une chaîne grasse.

2. Composition selon la revendication 1, dans laquelle R désigne un radical linéaire en C₁-C₃, notamment méthyle.

3. Composition selon l'une quelconque des revendications précédentes dans laquelle le dérivé C-glycoside est choisi parmi le C-β-D-xylopyranoside-2-hydroxy-propane et le C-α-D-xylopyranoside-2-hydroxy-propane, et est plus particulièrement le C-β-D-xylopyranoside-2-hydroxy-propane.

4. Composition selon l'une quelconque des revendications précédentes, comprenant d'environ 0,0001 % à environ 25 % en poids par rapport au poids total de la composition, et en particulier d'environ 0,001 % à environ 10 % en poids, et plus particulièrement d'environ 0,05 à 5 % en poids de matière active de dérivé C-glycoside par rapport au poids total de la composition.

5. Composition selon l'une quelconque des revendications précédentes, dans laquelle, le polymère associatif anionique est un terpolymère acrylique d'un acide carboxylique à insaturation α,β-éthylénique, d'un monomère uréthanne non-ionique qui est le produit de réaction d'un composé amphiphile non-ionique monohydrique avec un isocyanate à insaturation monoéthylénique.

6. Composition selon la revendication précédente, dans laquelle le terpolymère acrylique comprend, par rapport au poids total du terpolymère :
(a) environ de 20 à 70 % en poids, et de préférence de 25 à 55 % en poids, d'un acide carboxylique à insaturation α,β-éthylénique,
(b) environ de 20 à 80 % en poids, et de préférence de 30 à 65 % en poids, d'un monomère à insaturation éthylénique non tensioactif différent de (a), et
(c) environ de 0,5 à 60 % en poids, et de préférence de 10 à 50 % en poids, d'un monomère uréthanne non ionique qui est le produit de réaction d'un composé amphiphile non ionique monohydrique avec un isocyanate à insaturation monoéthylénique.

7. Composition selon l'une quelconque des revendications précédentes, **caractérisée en ce que** le polymère associatif est un terpolymère, et en particulier le terpolymère acide méthacrylique/acrylate de méthyle/diméthyl méta isopropényl benzyl isocyanate d'alcool béhényle éthoxylé.

8. Composition selon l'une quelconque des revendications précédentes, dans laquelle le polymère associatif est présent en une teneur variant de 0,01 à 30 % en poids, en particulier d'environ 0,05 à 15 % en poids, de préférence d'environ 0,1 à 10 % en poids et plus particulièrement d'environ 0,1 à 5 % en poids de polymère(s) associatif(s) par rapport au poids total de la composition.

9. Composition selon l'une quelconque des revendications précédentes, dans laquelle ladite composition comprend au moins une phase grasse.

10. Composition selon la revendication précédente, dans laquelle ladite phase grasse contient au moins un corps gras liquide à température ambiante et à pression atmosphérique et/ou au moins un corps gras solide à température ambiante et à pression atmosphérique.

11. Composition selon l'une quelconque des revendications précédentes, dans laquelle ladite composition se présente sous une forme fluide de type liquide vaporisable ou non, de pâte, d'émulsion directe ou inverse, ou de gel ou de support imprégné.

12. Composition selon l'une quelconque des revendications précédentes, dans laquelle ladite composition se présente sous une forme solide notamment compacte, pulvérulente ou coulée ou sous une forme de stick.

13. Composition selon l'une quelconque des revendications précédentes, dans lequel ladite composition se présente sous la forme d'un produit de soin, d'un produit solaire ou après solaire, de soin de photo-protection quotidienne, d'un produit pour le corps, d'un fond de teint à appliquer sur le visage ou le cou, d'un produit anti-cernes, d'un correcteur de teint, d'une crème teintée ou d'une base de maquillage pour le visage ou d'une composition de maquillage pour le corps.

14. Procédé de traitement non thérapeutique des matières et/ou des fibres kératiniques comprenant au moins l'étape d'appliquer sur lesdites matières et/ou fibres kératiniques au moins une couche d'une composition cosmétique telle que définie selon l'une quelconque des revendications 1 à 13.

## Claims

1. Cosmetic and/or pharmaceutical composition, especially a dermatological composition, comprising, in a physiologically acceptable medium containing an aqueous medium, at least one C-glycoside derivative and at least one associative polymer, said C-glycoside derivative corresponding to the general formula (I) below: 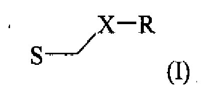 in which:
- R is a C₁-C₄ linear alkyl radical,
- X represents a -CH(OH)- group, and
- S represents D-xylose, and
- the bond S-CH₂-X represents a bond of C-anomeric nature, which may be α or β, and also the cosmetically acceptable salts thereof, the solvates thereof such as hydrates, and said associative polymer being chosen from:
- an anionic associative polymer chosen from the group consisting of:
- copolymers comprising at least one olefinic unsaturated carboxylic acid type hydrophilic radical, and at least one ester alkyl C₁₀-C₃₀ unsaturated carboxylic acid type hydrophobic radical, these polymers being **characterized in that** the olefinic unsaturated carboxylic acid type hydrophilic radical corresponds to the monomer of formula (II) below: 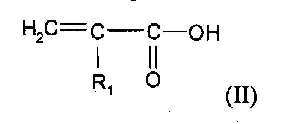
in which R₁ represents H or CH₃ or C₂H₅, for example acrylic acid, methacrylic acid or ethacrylic acid radicals,
and the ester alkyl C₁₀-C₃₀ unsaturated carboxylic acid type hydrophobic radical corresponds to the monomer of formula (III) below: 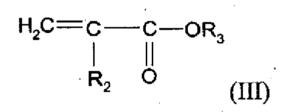 in which R₂ represents H or CH₃ or C₂H₅ (i.e. acrylate, methacrylate and ethacrylate radicals), and preferably H (acrylate radicals) or CH₃ (methacrylate radicals), R₃ represents an alkyl C₁₀-C₃₀ radical, preferably an alkyl C₁₂-C₂₂ radical,
- acrylic terpolymers obtained by reacting methacrylic acid (a), methyl acrylate (b), and non-ionic urethane macromonomer, being as represented below (III): 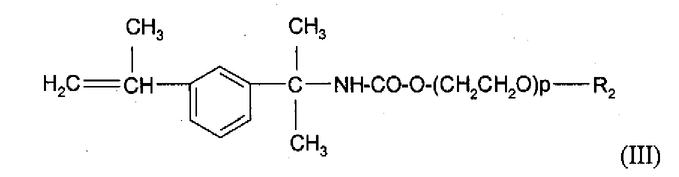
in which p is from 6 to 150 and R₂ is chosen from the group consisting of linear alkyl radicals comprising 18 to 26, and preferably 20 to 24 carbon atoms, the R₂ radical from the compound of formula (III) preferably being a radical from plant origin, such as the behenyl radical, and
- a non-ionic associative polymer selected from the group consisting of polyether-polyurethanes comprising hydrophilic blocks and at least one fatty chain.

2. Composition according to Claim 1, in which R denotes a linear C₁-C₃ radical, especially methyl.

3. Composition according to anyone of the preceding Claims, in which the C-glycoside derivative is chosen from C-β-D-xylopyranoside-2-hydroxypropane and C-α-D-xylopyranoside-2-hydroxypropane, and is more particularly C-β-D-xylopyranoside-2-hydroxypropane.

4. Composition according to anyone of the preceding Claims, comprising from about 0.0001% to about 25% by weight relative to the total weight of the composition, and in particular from about 0.001% to about 10% by weight, and more particularly from about 0.05% to 5%, of C-glycoside derivative active material relative to the total weight of the composition.

5. Composition according to anyone of the preceding Claims, in which the anionic associative polymer is an acrylic terpolymer of an α,β-ethylenically unsaturated carboxylic acid, and of a nonionic urethane monomer that is the product of reaction of a monohydric nonionic amphiphilic compound with a monoethylenically unsaturated isocyanate.

6. Composition according to the preceding claim, in which the acrylic terpolymer comprises, relative to the total weight of the terpolymer:
(a) from about 20% to 70% by weight, and preferably from 25 to 55% by weight, of an α,β-ethylenically unsaturated carboxylic acid,
(b) from about 20% to 80% by weight, and preferably from 30 to 65% by weight, of a non-surfactant ethylenically unsaturated monomer other than (a), and
(c) from about 0.5% to 60% by weight, and preferably from 10 to 50% by weight, of a nonionic urethane monomer that is the product of reaction of a monohydric nonionic amphiphilic compound with a monoethylenically unsaturated isocyanate.

7. Composition according to anyone of the preceding Claims, **characterized in that** the associative polymer is a terpolymer, and in particular the methacrylic acid/methyl acrylate/ethoxylated behenyl dimethyl-meta-isopropenyl benzyl isocyanate terpolymer.

8. Composition according to anyone of the preceding Claims, in which the associative polymer is present in a content ranging from 0.01% to 30% by weight, in particular from 0.05 to 15% by weight, preferably from 0.1 to 10% by weight and more particularly from 0.1 to 5% by weight, of associative polymer(s) relative to the total weight of the composition.

9. Composition according to anyone of the preceding Claims, in which the said composition comprises at least one fatty chain.

10. Composition according to the preceding claim, in which the said fatty phase contains at least one fatty substance that is liquid at room temperature and at atmospheric pressure and/or at least one fatty substance that is solid at room temperature and at atmospheric pressure.

11. Composition according to anyone of the preceding Claims, in which the said composition is in a fluid form of vaporizable or non-vaporizable liquid type, or in the form of a paste, a direct or inverse emulsion, a gel or an impregnated support.

12. Composition according to anyone of the preceding Claims, in which the said composition is in a solid form, especially a compact, pulverulent or cast form, or in the form of a stick.

13. Composition according to anyone of the preceding Claims, in which the said composition is in the form of a care product, an antisun product, an after-sun product, a daily photoprotective care product, a body care product, a foundation to be applied to the face or the neck, a concealer product, a complexion corrector, a tinted cream, a facial makeup base or a body makeup composition.

14. Non-therapeutic process for treating keratin materials and/or fibres, comprising at least the step of applying to the said keratin materials and/or fibres at least one coat of a cosmetic composition as defined according to anyone of Claims 1 to 13.

## Patentansprüche

1. Kosmetische und/oder pharmazeutische, insbesondere dermatologische Zusammensetzung, umfassend, in einem physiologisch verträglichen Medium, ein wässriges Medium, mindestens ein C-Glycosid-Derivat und mindestens ein assoziatives Polymer, wobei das C-Glycosid-Derivat der folgenden allgemeinen Formel (I) gehorcht: 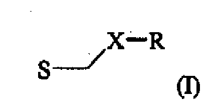 wobei:
R einen linearen C₁-C₄-Rest bezeichnet,
X eine Einheit -CH(OH)- bezeichnet, und
S D-Xylose bezeichnet, und
- die Bindung S-CH₂-X eine C-anomere Bindung darstellt, die α oder β sein kann, sowie ihre kosmetisch verträglichen Salze, ihre Solvate, wie die Hydrate, wobei das assoziative Polymer ausgewählt ist aus:
- einem anionischen assoziativen Polymer, das ausgewählt ist aus:
- Copolymeren, die mindestens eine hydrophile Einheit vom Typ einer ungesättigten olefinischen Carbonsäure und mindestens eine hydrophobe Einheit vom Typ eines ungesättigten Carbonsäure-(C₁₀-C₃₀)-alkylesters umfassen, wobei diese Polymere so sind, dass die hydrophile Einheit vom Typ einer ungesättigten olefinischen Carbonsäure dem Monomer der folgenden Formel (II) entspricht: 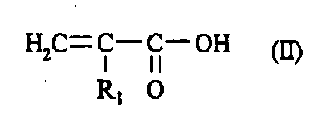 wobei R₁ H oder CH₃ oder C₂H₅ bezeichnet, beispielsweise Acrylsäure-, Methacrylsäure- oder Ethacrylsäure-Einheiten,
und wovon die hydrophobe Einheit vom Typ eines ungesättigten Carbonsäure-(C₁₀-C₃₀)-alkylesters dem Monomer der folgenden Formel (III) entspricht: 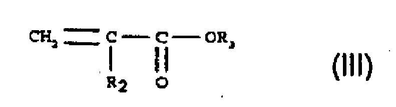 wobei R₂ H oder CH₃ oder C₂H₅ (d. h. Acrylat-, Methacrylat- oder Ethacrylat-Einheiten) und vorzugsweise H (Acrylateinheiten) oder CH₃ (Methacrylateinheiten) bezeichnet, R₃ einen C₁₀-C₃₀-Alkylrest und vorzugsweise einen C₁₂-C₂₂-Alkylrest bezeichnet,
- acrylischen Terpolymeren, die aus Methacrylsäure (a), Methylacrylat (b) und einem nichtionischen Urethan-Makromonomer (c) erhalten wurden, mit der folgenden Struktur (III): 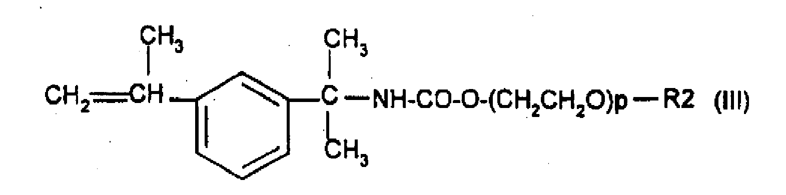 wobei p von 6 bis 150 reicht und R₂ ausgewählt ist aus linearen Alkylresten, die 18 bis 26 und vorzugsweise 20 bis 24 Kohlenstoffatome umfassen, der Rest R₂ in der Verbindung der Formel (III) vorzugsweise ein Rest pflanzlicher Herkunft ist, wie der Behenylrest, und
- einem nichtionischen assoziatives Polymer, das aus Polyether-Polyurethanen ausgewählt ist, das hydryophile Sequenzen und mindestens eine Fettsäurekette umfasst.

2. Zusammensetzung nach Anspruch 1, wobei R einen linearen C₁-C₃-Rest, insbesondere Methyl, bezeichnet.

3. Zusammensetzung nach einem der vorhergehenden Ansprüche, wobei das C-Gylcosid-Derivat aus C-β-D-Xylopyranosid-2-hydroxypropan und C-α-D-Xylopyranosid-2-hydroxypropan und insbesondere C-β-D-Xylopyranosid-2-hydroxypropan ausgewählt ist.

4. Zusammensetzung nach einem der vorhergehenden Ansprüche, umfassend etwa 0,0001 Gew.-% bis etwa 25 Gew.-%, bezogen auf das Gesamtgewicht der Zusammensetzung, und insbesondere etwa 0,001 Gew.-% bis etwa 10 Gew.-%, und spezieller etwa 0,05 Gew.-% bis 5 Gew.-% C-Glycosidderivat-Wirkstoff, bezogen auf das Gesamtgewicht der Zusammensetzung.

5. Zusammensetzung nach einem der vorhergehenden Ansprüche, wobei das anionische assoziative Polymer ein acrylisches Terpolymer ist von einer Carbonsäure mit α,β-ethylenischer Ungesättigtheit, einem nichtionischen Urethanmonomer, das das Reaktionsprodukt einer nichtionischen amphiphilen einbasigen Verbindung mit einem Isocyanat mit monoethylenischer Ungesättigtheit ist.

6. Zusammensetzung nach einem der vorhergehenden Ansprüche, wobei das acrylische Terpolymer, bezogen auf das Gesamtgewicht des Terpolymers, folgendes einschließt:
(a) etwa 20 bis 70 Gew.-% und vorzugsweise 25 bis 55 Gew.-% einer Carbonsäure mit α,β-ethylenischer Ungesättigtheit,
(b) etwas 20 bis 80 Gew.-%, und vorzugsweise 30 bis 65 Gew.-% eines von (a) verschiedenen nicht grenzflächenaktiven Monomers mit α,β-ethylenischer Ungesättigtheit, und
(c) etwa 0,5 bis 60 Gew.-% und vorzugsweise 10 bis 50 Gew.-% eines nichtionischen Urethanmonomers, das das Reaktionsprodukt einer nicht ionischen amphilen einbasigen Verbindung mit einem Isocyanat mit monethylenischer Ungesättigtheit ist.

7. Zusammensetzung nach einem der vorhergehenden Ansprüche, **dadurch gekennzeichnet, dass** das assoziative Polymer ein Terpolymer, und insbesondere das Terpolymer Methacrylsäure/Methylacrylat/Dimethyl-meta-isopropenylbenzylisocyanat von ethoxyliertem Behenylalkohol ist.

8. Zusammensetzung nach einem der vorhergehenden Ansprüche, wobei das assoziative Polymer in einem Gehalt vorhanden ist, der von 0,01 bis 30 Gew.-%, insbesondere von etwa 0,05 bis 15 Gew.-%, vorzugsweise von etwa 0,1 bis 10 Gew.-% und spezieller von etwa 0,1 bis 5 Gew.-% von assoziativem(en) Polymer(en), bezogen auf das Gesamtgewicht der Zusammensetzung, variiert.

9. Zusammensetzung nach einem der vorhergehenden Ansprüche, wobei die Zusammensetzung mindestens eine Fettphase umfasst.

10. Zusammensetzung nach einem der vorhergehenden Ansprüche, wobei die Fettphase mindestens einen bei Umgebungstemperatur und bei Atmosphärendruck flüssigen Fettkörper und/oder mindestens einen bei Umgebungstemperatur und bei Atmosphärendruck festen Fettkörper enthält.

11. Zusammensetzung nach einem der vorhergehenden Ansprüche, wobei die Zusammensetzung in einer fluiden Form vom Typ einer verdampfbaren oder nicht verdampfbaren Flüssigkeit, einer Paste, einer direkten oder inversen Emulsion, oder einem Gel oder imprägnierten Träger vorliegt.

12. Zusammensetzung nach einem der vorhergehenden Ansprüche, wobei die Zusammensetzung in einer festen, insbesondere kompakten, feinpulvrigen oder gegossenen Form oder in der Form eines Sticks dargereicht wird.

13. Zusammensetzung nach einem der vorhergehenden Ansprüche, wobei die Zusammensetzung in der Form eines Pflegeprodukts, eines Sonnenschutz- oder After-sun-Produkts, eines Lichtschutzprodukts zur täglichen Pflege, eines Produkts für den Körper, einer Teintgrundierung zum Auftragen auf das Gesicht oder den Hals, eines Antifaltenprodukts, eines Teint-Korrekturprodukts, einer getönten Creme oder einer Schminkgrundlage für das Gesicht oder einer Schminkzusammensetzung für den Körper vorliegt.

14. Verfahren zur nicht therapeutischen Behandlung von keratinischen Substanzen oder keratinischen Fasern, das mindestens einen Schritt des Aufbringens auf die keratinischen Substanzen oder keratinischen Fasern, von mindestens einer Schicht von einer kosmetischen Zusammensetzung, wie nach einem der Ansprüche 1 bis 13 definiert, umfasst.
